# EUROPÄISCHE PATENTSCHRIFT

(11) **EP 0 750 501 B1**
(45) Veröffentlichungstag und Bekanntmachung des Hinweises auf die Patenterteilung: **21.08.2002**
(21) Anmeldenummer: 95913171.5
(22) Anmeldetag: 30.03.1995
(51) Int. Cl.: A61K 31/57, A61K 31/565

(54) **PHARMAZEUTISCHES KOMBINATIONSPRÄPARAT ZUR HORMONALEN KONTRAZEPTION**
COMBINED HORMONAL CONTRACEPTION PHARMACEUTICAL PREPARATION
PREPARATION PHARMACEUTIQUE COMBINEE DE CONTRACEPTION HORMONALE

(30) Priorität: 30.03.1994 DE 4411585
(43) Veröffentlichungstag der Anmeldung: 02.01.1997
(73) Patentinhaber: SCHERING AKTIENGESELLSCHAFT, 13353 Berlin (DE)
(72) Erfinder: LACHNIT, Ursula, D-10715 Berlin (DE); DÜSTERBERG, Bernd, D-12307 Berlin (DE); SPONA, Jürgen, A-1190 Wien (AT)
(86) Internationale Anmeldenummer: EP9501190
(87) Internationale Veröffentlichungsnummer: WO95026730

(56) Entgegenhaltungen:
- EP-A- 0 499 348

## Beschreibung

Die vorliegende Erfindung betrifft ein pharmazeutisches Kombinationspräparat mit zwei in einer Verpackungseinheit räumlich getrennt konfektionierten, zur zeitlich sequentiellen oralen Verabreichung bestimmten Hormonkomponenten, die jeweils aus einer Anzahl räumlich getrennt und einzeln entnehmbar in der Verpackungseinheit untergebrachten täglichen Dosierungseinheiten bestehen, wobei eine erste der Hormonkomponenten als hormonellen Wirkstoff in Kombination ein Estrogen- und in mindestens zur Ovulationshemmung ausreichender Dosierung ein Gestagenpräparat in entweder ein- oder mehrphasiger Ausbildung und die zweite Hormonkomponente als hormonellen Wirkstoff lediglich ein Estrogenpräparat enthält, wobei die erste Hormonkomponente 24 und die zweite Hormonkomponente 4 bis 8 Tageseinheiten umfaßt, die Tageseinheiten der ersten Hormonkomponente nicht die Kombination eines biogenen Estrogens und eines synthetischen Estrogens enthalten, und die Gesamtzahl der Hormontageseinheiten gleich der Gesamtzahl der Tage des gewünschten, mindestens aber 28 Tage langen, Zyklus ist, sowie eine entsprechende, dieses Kombinationspräparat enthaltende Packung.

Orale Kontrazeptiva in Form von Kombinationspräparaten sind als sogenannte Einphasenpräparate seit 1960 bekannt. Diese Präparate bestehen aus 21 wirkstoffhaltigen Dosierungseinheiten und 7 wirkstofffreien Tabletten oder Dragees. Die tägliche Dosierungseinheit ist aus einem Estrogen und Gestagen zusammengesetzt. In Einphasenpräraraten ist die täglich zu verabreichende Dosis der aktiven Stoffe in jeder Dosierungseinheit gleich hoch. Wenn die täglich zu verabreichende Dosis der aktiven Bestandteile in den einzelnen Dosierungseinheiten in einzelnen Abschnitten über den Verabreichungszyklus unterschiedlich ist, handelt es sich um sogenannte Mehrphasenpräparate. Als besonders namhafter Vertreter sei Triquilar® genannt (DE-A 23 65 103).

Durch die Entwicklung neuer, wirksamerer Gestagene als die in den ersten oralen Kontrazeptiva enthaltenen, konnte die tägliche Gestagendosierung kontinuierlich verringert werden. Auch die tägliche Estrogendosierung konnte gesenkt werden, obwohl als Estrogen in hormonalen Kontrazeptiva nach wie vor meist Ethinylestradiol enthalten ist.

Bei der Entwicklung neuer, verbesserter oraler Kontrazeptiva standen (und stehen) folgende drei Gesichtspunkte im Vordergrund:
Es soll
(1) die kontrazeptive Sicherheit,
(2) eine gute Zykluskontrolle, d.h. geringe Inzidenz an Zwischenblutungen und
(3) ein Minimum an unerwünschten Nebenwirkungen gewährleistet sein.
Die kontrazeptive Sicherheit wird vor allem durch die Gestagenkomponente bewirkt. Deren tägliche Dosierungsmenge enspricht jeweils mindestens der Grenzdosis, die für das betreffende Gestagen zur Ovulationshemmung als erforderlich angesehen wird. Das in Kombinationspräparaten als Estrogen meistens verwendete Ethinylestradiol soll den ovulationshemmenden Effekt des Gestagens erhöhen und vor allem die Zyklusstabilität gewährleisten. Die tägliche Dosis bei alleiniger Verabreichung des Ethinylestradiols, die für eine Hemmung der Ovulation verwendet werden muss, beträgt 100µg.

Kombinationspräparate mit der jüngsten Generation von Gestagenen sind z.B. die Einphasenpräparate Femovan (DE-PS 2546062) oder Marvelon (DE-OS 2361120).
Als Mehrphasenpräparat, dessen Dosierungseinheiten ein Gestagen der jüngsten Generation, nämlich Gestoden, enthalten, ist beispielsweise Milvane® zu nennen (EP- 0 148 724). Bei diesen Dreiphasenpräparaten werden in der ersten Phase zumeist 4-6 Dragees verabreicht, in der jedes Dragee eine Estrogenmenge in geringer Dosis und ein Gestagen in geringer Dosis enthält. In der zweiten Phase von 4-6 Dragees enthält jede Dosierungseinheit ein Estrogen mit gleicher oder gering angehobener, maximal bis auf das 2 fache gesteigerte Dosis und ein Gestagen mit gleicher oder gering angehobener, maximal auf das 1.5 fach gesteigerte Dosis. In einer dritten Phase von 9-11 Einheiten enthält jedes Dragee ein Estrogen mit gleicher oder wieder gesenkter, maximal auf den Ausgangswert erniedrigter Dosis und ein Gestagen mit weiter angehobener, maximal auf das 3 fache des Ausgangswertes gesteigerter Dosis. Daran schliessen sich 7 pillenfreie Tage an.
Neuerdings sind auch mehrphasische Kombinationspräparate vorgeschlagen worden, die eine verlängerte, d.h. bis zu 24tägige Einnahme wirkstoffhaltiger Dosierungseinheiten im 28tägigen Zyklus vorsehen können. Dabei steigt die tägliche Gestagen-Dosierungsmenge von der ersten über die zweite bis zur dritten Phase entweder an (EP-A 0 491 415) oder sie nimmt ab (EP-A 0 491 438). Zur Vervollständigung des 28 Tage langen Zyklus schließen sich im ersten Fall 4 Blindpillentage, 4 Placebos oder aber 4 ausschließlich gestagenhaltige Dosierungseinheiten oder im zweiten Fall 4 bis 7 Blindpillentage oder 4 bis 7 Placebos an.

Die Entwicklung neuer oraler Kontrazeptiva mit verringerter täglicher Hormondosis hatte zum Ziel, die in epidemologischen Studien beschriebenen Nebenwirkungen zu minimieren. Neuere epidemologische Daten weisen auf einen solchen Trend zur besseren Verträglichkeit niedriger dosierter Präparate hinsichtlich kardiovaskulärer Nebenwirkungen hin.[ Thorogood M, Oral Contraceptives and Cardiovascular Disease: An Epidemiologic Overview; Pharmacoepidemiology and Drug Safety, Vol 2: 3-16 (1993); Gerstman B B, Piper J M, Tomita D K, Ferguson W J, Stadel B V, Lundin F E; Oral Contraceptive Estrogen Dose and the Risk of Deep Venous Thromboembolic Disease, Am J E, Vol. 133, No 1, 32-36 (1991); Lidegaard O, Oral contraception and rist of a cerebral thromboembolic attack: results of a case-control study; BMJ Vol 306, 956-63 (1993); Vessey M, Mant D, Smith A, Yeates D., Oral contraceptives and venous thromboembolism: findings in a large prospective study; BMJ, Vol 292, (1986); Mishell D R, Oral Contraception: Past, Present, and Future Perspectives; Int J Fertil, 36 Suppl., 7 - 18 (1991)].

Ein Zusammenhang zwischen der Höhe der täglichen Estrogendosis und der Häufigkeit kardiovaskulärer Komplikationen wird angenommen.

Das Präparat mit der zur Zeit niedrigst dosierten Estrogenmenge ist als Mercilon® im Handel und enthält 20µg Ethinylestradiol in Kombination mit 150µg Desogestrel in jeder täglichen Dosierungseinheit über 21 Tage mit einem sich anschließenden 7 tägigem pillenfreien Intervall. Die Zykluskontrolle dieses Präparates ist im Vergleich zu Präparaten mit höherer Estrogendosis erwartungsgemäß etwas schlechter. Ein weiteres, klinisch bedeutsames Problem stellt die in mehreren Studien übereinstimmend gemachte Beobachtung einer geringeren ovariellen Suppression des 20µg Ethinylestradiol enthaltenden Präparates dar. Es kommt offensichtlich unter dieser sehr niedrigen Estrogendosis bei vielen Frauen zur Heranreifung von Follikeln, die mit Ultraschalluntersuchungen bzw. Hormonuntersuchungen nachgewiesen werden konnten [Lunell N O, Carlström K, Zador G, Ovulation inhibition with a combined oral contraceptive containing 20µg ethinylestradiol and 250µg levonorgestrel; Acta Obstet Gynecol Scand Suppl. 88: 17-21 (1979); Mall-Haefeli M, Werner-Zodrow I, Huber P R, Klinische Erfahrungen mit Mercilon und Marvelon unter besonderer Berücksichtigung der Ovar-Funktion; Geburtsh. und Frauenheilk. 51, 35-38, Georg Thieme Verlag, Stuttgart-New York (1991); Strobel E, Behandlung mit oralen Kontrazeptiva; Fortschr. Med. 110 Jg. Nr. 20 (1992); Letter to Editor, Contraception 45: 519-521 (1992); Teichmann A T, Brill K, Can Dose Reduction of Ethinylestradiol in OCs jeopardize Ovarian Suppression and Cycle Control? Abstract Book, VIIIth World Congress on Human Reproduction, Bali, Indonesia (1993)].

Bis vor kurzem wurde eine mehrtägige Unterbrechung der Einnahme wirkstoffhaltiger Dragees für notwendig gehalten, um eine Entzugsblutung auszulösen und eine ausreichende Zykluskontrolle zu gewährleisten.

Andere Präparate wurden beschrieben, die einen estrogenen und gestagenen Wirkstoff enthalten und die im allgemeinen über 21 Tage in gleichbleibenden Mengen in jeder einzelnen Dosierungseinheit verabreicht werden, bei denen der Einnahme dieser einen estrogenen und gestagenen Wirkstoff enthaltenden Dosierungseinheiten die Einnahme ausschließlich Estrogen-haltiger Dosierungseinheiten (Ijzerman, US-A 3,502,772; Pasquale, US-A 4,921,843; Kuhl et al., EP-A 0 499 348) vorausgeht. Bei diesen Präparaten wird bei Einnahmebeginn entweder bereits am ersten Zyklustag (Kuhl) oder frühestens am zweiten Zyklustag (Pasquale) mit der Einnahme von Dosierungseinheiten begonnen, die nur einen estrogenen Wirkstoff enthalten, und zwar in einer Dosierung, die unter der ovulationshemmenden Dosis der estrogenen Komponente liegt, wodurch es zu Follikelentwicklungen kommen kann. Follikelentwicklungen werden für Durchbruchsovulationen verantwortlich gemacht (Chowdhury et al., "Escape" ovulation in women due to the missing of low dose combination oral contraceptive pills, Contraception, 22: 241-247, 1980; Molloy B.G. et al., "Missed pill" conception: fact or fiction? Brit.Med.J. 290, 1474-1475, 1985). Der kontrazeptive Schutz ist dadurch in Frage gestellt. Das Risiko einer Schwangerschaft ist daher insbesondere bei Einnahmefehlern unter den 20µg-Ethinylestradiol Präparaten hoch.

Aus der DE-PS 43 08 406 (nicht-vorveröffentlicht) ist bereits ein ovulationshemmendes Mittel in Form eines Kombinationspräparates zur Kontrazeption bekannt, bei dem zumindest eine sowohl estrogen- als auch gestagenhaltige Hormonkomponente vorgesehen ist, bei der die Tageseinheiten sowohl ein biogenes Estrogen als auch ein synthetisches Estrogen enthalten. Die vorliegende Erfindung bezieht sich nicht auf derartige Präparate.

Die Aufgabe der vorliegenden Erfindung ist es, ein Kombinationspräparat mit möglichst niedrigem Estrogengehalt in jeder einzelnen Dosierungseinheit aber auch mit einem niedrigen Gesamthormongehalt pro Verabreichungszyklus zur Verfügung zu stellen, wobei bei hoher kontrazeptiver Sicherheit eine möglichst geringe Inzidenz an Follikelentwicklung, eine einwandfreie Zykluskontrolle unter zuverlässiger Vermeidung von Zwischenblutungen wie Durchbruchblutungen und "spottings" erreicht und unerwünschte Nebenwirkungen vermieden werden sollen.

Diese Aufgabe wird durch die Bereitstellung des eingangs angegebenen zweiphasischen Kombinationspräparates gelöst.

In der ersten Phase wird beginnend mit dem ersten Zyklustag eine Dosierungseinheit enthaltend ein Estrogen in Kombination mit einer gestagenen Komponente täglich über 24 Tage verabreicht. Daran schliesst sich die zweite Phase an, in der über den verbleibenden Zeitraum zum 28 Tage umfassenden Zyklus über 4 Tage ein Estrogen verabreicht wird.

Es werden 24 tägliche ein Estrogen- und ein Gestagenpräparat enthaltende Dosierungseinheiten sowie 4 tägliche ausschließlich ein Estrogenpräparat enthaltende Dosierungseinheiten verabreicht.

Die erste, sowohl estrogen- als auch gestagen-haltige Phase kann dabei auch in dem Fachmann geläufiger Weise mehrphasig, beispielsweise triphasig, ausgebildet sein (siehe hierzu beispielsweise EP-A 0 148 724). Ein solches Präparat ist dann als Vierphasen-Präparat zu bezeichnen.
Die einphasige Ausbildung der ersten Hormonkomponente ist jedoch bevorzugt.

Bei Einnahme des erfindungsgemäßen Kombinationspräparates wird bereits im ersten Verabreichungszyklus die Rekrutierung des dominanten Follikels, die im spontanen Zyklus während der ersten 6 Tage des Menstruationszyklus erfolgt, effizient unterdrückt. Somit lassen sich mit dem Kombinationspräparat der vorliegenden Erfindung die Follikelentwicklung bereits im ersten Einnahmezyklus unterdrücken und damit Durchbruchsovulationen vermeiden, wodurch die kontrazeptive Sicherheit erhöht wird.
Dies ist vor allem bei Einnahmefehlern von eminenter Wichtigkeit, und zwar insbesondere bei hormonalen Kontrazeptiva mit niedriger täglicher Ethinylestradiol-Dosismenge. Da bei 25% der Frauen, die die Pille nehmen, Einnahmefehler (Auslassen von Dosierungseinheiten oder Verlängerung des Intervalls auf über 24 Stunden zwischen der täglichen Einnahme zweier Dosierungseinheiten) bekannt sind (Finlay I.G., Scott M.B.G.: Patterns of contraceptive pilltaking in an inner city practice. Br.Med.J. 1986, 293: 601-602), erhöht das erfindungsgemäße Kombinatioinspräparat, wenn es als ovulationshemmendes Mittel verwendet wird, die kontrazeptive Sicherheit. Dies trifft insbesondere bei niedrigst dosierten Präparaten zu.
Die Erhöhung der Anzahl von Dosierungseinheiten über die übliche Zahl von 21 Tagen auf 24 Tage bewirkt eine effektvolle Verkürzung des pillenfreien Intervalls, in dem bei konventionellen Kombinationspräparaten wie in einem normalen Menstruationszyklus die Selektion von Follikeln erfolgt und somit eine Follikelentwicklung entsteht und vermehrt endogenes Estrogen gebildet wird. Diese Follikel führen, wie schon oben ausgeführt, zu Durchbruchsovulationen. Besonders bei Einnahmefehlern treten diese Durchbruchsovulationen vermehrt auf.
Die anschließende Phase, in der über 4 bis 8 Tage Dosierungseinheiten täglich verabreicht werden, die nur eine estrogene Komponente als hormonalen Wirkstoff enthalten, gewährleistet eine Abbruchsblutung und bewirkt eine Stimulierung von Progesteronrezeptoren im Endometrium, wodurch im folgenden Verabreichungszyklus eine verringerte Zwischenblutungsrate verglichen mit herkömmlichen, niedrig dosierten Präparaten erreicht wird.

Gemäß einer bevorzugten Ausführungsform der Erfindung wird das Estrogen der ersten Hormonkomponente ausgewählt aus der Gruppe der Verbindungen
17β-Estradiol,
Ethinylestradiol und
17β-Estradiolvalerat
und das Gestagen ausgewählt aus der Gruppe der Verbindungen
Gestoden,
Levonorgestrel,
Desogestrel,
3-Ketodesogestrel,
Drospironenon,
Cyproteronacetat,
Norgestimat und
Norethisteron sowie
das Estrogen der zweiten Hormonkomponente ausgewählt aus der Gruppe der Verbindungen.
17β-Estradiol,
Ethinylestradiol und
17β-Estradiolvalerat.

Gemäß einer weiteren bevorzugten Variante der vorliegenden Erfindung ist das Estrogen der ersten Hormonkomponente in jeder täglichen Dosierungseinheit in einer Dosis von
1.0 bis 6.0 mg 17β-Estradiol,
0.01 bis 0.04 mg Ethinylestradiol,
1.0 bis 6.0 mg 17β-Estradiolvalerat
und das Gestagen in jeder täglichen Dosierungseinheit in einer Dosis von
0.04 bis 0.075 mg Gestoden,
0.05 bis 0.125 mg Levonorgestrel,
0.06 bis 0.15 mg Desogestrel,
0.06 bis 0.15 mg 3-Ketodesogestrel,
1.0 bis 3.0 mg Drospironenon,
1.0 bis 2.0 mg Cyproteronacetat,
0.2 mg bis 0.3 mg Norgestimat,
0.35 bis 0.75 mg Norethisteron
enthalten.

Für Ethinylestradiol sind 0,015 bis 0,025 mg, für 17β-Estradiolvalerat 1,0 bis 4,0 mg und für Gestoden 0,05 bis 0,075 mg als tägliche Menge in den Tageseinheiten der ersten Hormonkomponente besonders bevorzugt.

Die zweite Hormonkomponente enthält das Estrogen in jeder täglichen Dosierungseinheit vorzugsweise in einer Menge von
1.0 bis 6.0 mg 17β-Estradiol,
0.002 bis 0.04 mg Ethinylestradiol,
1.0 bis 6.0 mg 17β-Estradiolvalerat.

Gemäß einer besonders bevorzugten Ausführungsform enthält die zweite Hormonkomponente in jeder täglichen Dosierungseinheit als Estrogen Ethinylestradiol in einer Menge von 0.01 bis 0.025 mg, 17β-Estradiol in einer Menge von 1,0 bis 3,0 mg oder 17β-Estradiolvalerat in einer Menge von 1,0 bis 4,0 mg.

Ein Präparat gemäß vorliegender Erfindung enthält insgesamt vorzugsweise 28 Hormontageseinheiten.

Als Estrogen für die erste sowohl als auch die zweite Hormonkomponente kommt in erster Linie Ethinylestradiol oder 17β-Estradiol in Betracht.

Von den genannten Gestagenen für die zweite Hormonkomponente ist Gestoden hervorzuheben; auch Levonorgestrel ist bevorzugt.

17β-Estradiolvalerat, welches als Estrogen sowohl in der ersten als auch in der zweiten Hormonkomponente enthalten sein kann, ist nur als ein möglicher Vertreter dieser 17β-Estradiolester genannt; auch andere derartige, homologe Ester können als estrogene Komponente im Rahmen der vorliegenden Erfindung verwendet werden.

Das nachfolgende Beispiel dient der näheren Erläuterung der vorliegenden Erfindung:

### Beispiel 1:

| | | | | | | | |
|---|---|---|---|---|---|---|---|
| Tag | 1 | 2 | 3 | 4 | 5 | 6 | 7 |
| Zusammensetzung | C | C | C | C | C | C | C |
| | | | | | | | |
| Tag | 8 | 9 | 10 | 11 | 12 | 13 | 14 |
| Zusammensetzung | C | C | C | C | C | C | C |
| | | | | | | | |
| Tag | 15 | 16 | 17 | 18 | 19 | 20 | 21 |
| Zusammensetzung | C | C | C | C | C | C | C |
| | | | | | | | |
| Tag | 22 | 23 | 24 | 25 | 26 | 27 | 28 |
| Zusammensetzung | C | C | C | E | E | E | E |
| Tag = Tag des Menstruationszyklus, Tag 1 ist der erste Blutungstag | | | | | | | |
| C = Kombination von Estrogen und Gestagen (= erste Hormonkomponente) | | | | | | | |
| E = Estrogen (= zweite Hormonkomponente). | | | | | | | |

Die Formulierung der Dosierungseinheiten erfolgt konventionell unter Verwendung von für die Herstellung Estrogen-/Gestagen- sowie ausschließlich Estrogen-haltiger Tabletten, Pillen, Dragees, etc. bekannter Hilfsstoffe.

Die Anzahl der Tageseinheiten in den einzelnen Phasen innerhalb der ersten Hormonkomponente im Falle der Ausbildung des erfindungsgemäßen Kombinationspräparates als Vierphasen-Präparat ist nachfolgend angegeben:

| | 1. Phase | 2. Phase | 3. Phase | 4. Phase |
|---|---|---|---|---|
| Anzahl der Tageseinheiten | 4 bis 9 C1 | 4 bis 9 C2 | 9 bis 13 C3 | E = 28-(C1+C2+C3) |
| C1, C2, C3 = Kombination Gestagen und Estrogen innerhalb der ersten Hormonkomponente | | | | |

Erfindungsgemäß sollen die Dosierungsverhältnisse Estrogen/Gestagen in den täglichen Dosierungseinheiten der einzelnen Phasen innerhalb der nachstehend angegebenen Bereiche liegen. Die Dosierung in der 1. Phase ist hierbei als 1 gesetzt und die Dosisbereiche in den folgenden Phasen sind als Mehrfaches der Dosierung in der 1. Phase angegeben:

| | 1. Phase | 2. Phase | 3. Phase | 4. Phase |
|---|---|---|---|---|
| Estrogen | 1 | 1 - 2 | 0,5 - 1,5 | 0,5 - 1 |
| Gestagen | 1 | 1 - 1,5 | 1,5 - 3 | ------ |

Die Zusammensetzung eines erfindungsgemäßen Kombinationspräparates in vierphasiger Ausbildung ist dem nachfolgenden Beispiel zu entnehmen:

| | 1. Phase | 2. Phase | 3. Phase | 4. Phase |
|---|---|---|---|---|
| Anzahl der | 5 C1 | 7 C2 | 12 C3 | 4 E |
| Tageseiheiten | | | | |
| Ethinylestradiol [mg] | 0,020 | 0,025 | 0,020 | 0,010 |
| oder | | | | |
| 17β-Estradiol [mg] | 2,000 | 3,000 | 2,000 | 1,000 |
| **und** | | | | |
| Gestoden [mg] | 0,050 | 0,060 | 0,070 | ------ |
| oder | | | | |
| Levonorgestrel [mg] | 0,050 | 0,075 | 0,100 | ------ |

Das erfindungsgemäße Kombinationspräparat dient der Empfängnisverhütung für die Frau durch Verabreichung der täglichen Dosierungseinheiten der ersten Hormonkomponente über 24 Tage, beginnend am Tag eins des Menstruationszyklus (erster Tag der Menstruationsblutung), gefolgt von 4 bis 8 täglichen Dosierungseinheiten, die ausschließlich ein Estrogen (E) enthalten, während insgesamt mindestens 28 Tagen im Verabreichungszyklus. Mit diesem Kombinationspräparat kann eine ausgeprägte ovarielle Suppression ohne häufige Follikelanreifung sowie hervorragender Zykluskontrolle bei niedriger täglicher Estrogendosierung, niedriger Gesamtestrogen- sowie niedriger Gesamthormonmenge pro Verabreichungszyklus erreicht werden.

Die Vorteile dieses über im allgemeinen 28 Tage verabreichten, erfindungsgemäßen Kombinationspräparates (ovulationshemmendes Mittel) gegenüber den bisher beschriebenen Präparaten, insbesondere denen mit einer täglichen Ethinylestradiol-Dosis von weniger als 30µg und solchen mit pillenfreiem Intervall, lassen sich wie folgt charakterisieren:
1. Eine signifikant geringere Häufigkeit von Follikelentwicklungen bei der Anwenderin. Dies bedeutet eine geringere Gefahr von Durchbruchovulationen und damit eine grössere kontrazeptive Zuverlässigkeit insbesondere bei Einnahmefehlern.
2. Die Rekrutierung des dominanten Follikels wird durch die Verlängerung der Einnahme der Kombination auf 24 Tage bereits im ersten Zyklus unterdrückt.
3. Die Einnahme von je 4 bis 8 täglichen Estrogen-Dosierungseinheiten im Anschluss an die Verabreichung der 24 tägigen Kombinationsdosierung führt zu einer deutlich verbesserten Zykluskontrolle und zu einer geringeren Inzidenz von Nebenwirkungen wie Kopfschmerzen im Rahmen des prämenstruellen Syndroms.
4. Andere klinische Symptome, die auf stark fluktuierende endogene Estrogenspiegel zurückzuführen sind, wie beispielsweise Brustspannen, sind aufgrund der bedeutend stärkeren ovariellen Supprimierung ebenfalls deutlich 0,015 bis 0,025 mg verringert.

Die vorstehend genannten Vorteile, insbesondere die Supprimierung der Follikelentwicklung und die damit einhergehende Inhibierung der endogenen Estrogenproduktion, sind bei den erfindungsgemäßen Kombinationspräparaten, besonders ausgeprägt.

Die Formulierung eines Estrogens und Gestagens für die Herstellung eines erfindungsgemäßen Kombinationspräparates erfolgt vollkommen analog wie es bereits für herkömmliche orale Kontrazeptiva mit 21tägiger Einnahmedauer der Wirkstoffe, wie beispielsweise Femovan® (Ethinylestradiol/Gestoden) oder Microgynon® (Ethinylestradiol/Levonorgestrel) bekannt ist. Die Formulierung der ausschließlich Estrogenhaltigen Dosierungseinheiten kann ebenso ganz analog wie es für schon erhältliche, zur oralen Anwendung bestimmten Estrogen-haltige Mittel, beispielsweise ProgynonC®, bekannt ist, durchgeführt werden.
Eine ein erfindungsgemäßes Kombinationspräparat enthaltende Packung ist ebenfalls analog wie Packungen für bereits bekannte, am Markt befindliche orale Kontrazeptiva aufgebaut, mit der Abweichung, daß anstelle der üblichen 21, die aktiven Bestandteile enthaltenden, Dosierungseinheiten, nunmehr 24 derartige Dosierungseinheiten und weitere 4 bis 8, lediglich Estrogen-haltige Dosierungseinheiten, vorhanden sind. Als Verpackungsform für das erfindungsgemäße Kombinationspräparat dient im allgemeinen eine herkömmliche Blisterpackung, jedoch sind auch andere für diesen Zweck bekannte Verpackungsformen denkbar.

Die Erfindung betrifft außerdem auch ein Verfahren zur weiblichen Kontrazeption bei dem das vorstehend beschriebene Kombinationspräparat in der angegebenen Weise verabreicht wird.

Zur Bestimmung wirkequivalenter Mengen von Ethinylestradiol und 17β-Estradiol einerseits und verschiedener Gestagene wie Gestoden, Levonorgestrel, Desogestrel und 3-Ketodesogestrel andererseits wird auf die in der EP-A- 0 253 607 gemachten Angaben verwiesen. Weitere Einzelheiten zur Bestimmung von Dosisequivalenten verschiedener gestagener Wirkstoffe finden sich beispielsweise in "Probleme der Dosisfindung: Sexualhormone"; F. Neumann et al. in "Arzneimittelforschung" (Drug Research) 27, 2a, 296 - 318 (1977) sowie in "Aktuelle Entwicklungen in der homonalen Kontrazeption"; H. Kuhl in "Gynäkologe" 25: 231 - 240 (1992).

## Patentansprüche

1. Pharmazeutisches Kombinationspräparat mit zwei in einer Verpackungseinheit räumlich getrennt konfektionierten, zur zeitlich sequentiellen oralen Verabreichung bestimmten Hormonkomponenten, die jeweils aus einer Anzahl räumlich getrennt und einzeln entnehmbar in der Verpackungseinheit untergebrachten täglichen Dosierungseinheiten bestehen, wobei eine erste der Hormonkomponenten als hormonellen Wirkstoff in Kombination ein Estrogen- und in mindestens zur Ovulationshemmung ausreichender Dosierung ein Gestagenpräparat in entweder ein- oder mehrphasiger Ausbildung und die zweite Hormonkomponente als hormonellen Wirkstoff lediglich ein Estrogenpräparat enthält, wobei die erste Hormonkomponente 24 und die zweite Hormonkomponente 4 bis 10 Tageseinheiten umfaßt, die Tageseinheiten der ersten Hormonkomponente nicht die Kombination eines biogenen Estrogens und eines synthetischen Estrogens enthalten, und die Gesamtzahl der Hormontageseinheiten gleich der Gesamtzahl der Tage des gewünschten, mindestens aber 28 Tage langen, Zyklus ist.

2. Kombinationspräparat nach Anspruch 1, **dadurch gekennzeichnet, daß** das Estrogen der ersten Hormonkomponente aus der Gruppe der Verbindungen
17β-Estradiol,
Ethinylestradiol und
17β-Estradiolvalerat
und das Gestagen aus der Gruppe der Verbindungen
Gestoden,
Levonorgestrel,
Desogestrel,
3-Ketodesogestrel,
Drospironenon,
Cyproteronacetat,
Norgestimat und
Norethisteron sowie
das Estrogen der zweiten Hormonkomponente aus der Gruppe der Verbindungen
17β-Estradiol,
Ethinylestradiol und
17β-Estradiolvalerat
ausgewählt ist.

3. Kombinationspräparat nach Anspruch 2, **dadurch gekennzeichnet, daß** das Estrogen der ersten Hormonkomponente in jeder täglichen Dosierungseinheit in einer Dosis von
1.0 bis 6.0 mg 17β-Estradiol,
0.015 bis 0.025 mg Ethinylestradiol,
1.0 bis 4.0 mg 17β-Estradiolvalerat
und das Gestagen in jedcr täglichen Dosierungseinheit in einer Dosis von
0.05 bis 0.075 mg Gestoden
0.05 bis 0.125 mg Levonorgestrel
0.06 bis 0.15 mg Desogestrel
0.06 bis 0.15 mg 3-Ketodesogestrel
1.0 bis 3.0 mg Drospironenon
1.0 bis 2.0 mg Cyproteronacetat
0.2 mg bis 0.3 mg Norgestimat
0.35 bis 0.75 mg Norethisteron
enthalten ist.

4. Kombinationspräparat nach Anspruch 2 oder 3, **dadurch gekennzeichnet, daß** in der zweiten Hormonkomponente in jeder täglichen Dosierungseinheit eine Menge von
1.0 bis 6.0 mg 17β-Estradiol,
0.002 bis 0.04 mg Ethinylestradiol,
1.0 bis 4.0 mg 17β-Estradiolvalerat.

5. Kombinationspräparat nach Anspruch 4, **dadurch gekennzeichnet, daß** in der zweiten Hormonkomponente in jeder täglichen Dosierungseinheit Ethinylestradiol in einer Menge von 0.01 bis 0.025 mg enthalten ist.

6. Kombinationspräparat nach einem der vorhergehenden Ansprüche, **dadurch gekennzeichnet, daß** die Gesamtzahl der Hormontageseinheiten 28 beträgt.

## Claims

1. Pharmaceutical combination preparation with two hormone components that are packaged spatially separately in a packaging unit and that are intended for time-sequential oral administration, which in each case consist of a number of daily dosage units that are placed spatially separately and individually removably in the packaging unit, where as a hormonal active ingredient a first hormone component contains in combination an estrogen preparation and in at least a dosage that is sufficient to inhibit ovulation a progestogen preparation in either a one-phase or multiple-phase form and as a hormonal active ingredient the second hormone component contains only an estrogen preparation, where the first hormone component comprises 24 daily units and the second hormone component comprises 4 to 10 daily units, the daily units of the first hormone component do not contain the combination of a biogenic estrogen and a synthetic estrogen, and the total number of hormone daily units is equal to the total number of days of the desired cycle, but at least 28 days in length.

2. Combination preparation according to Claim 1, **characterized in that** the estrogen of the first hormone component is selected from the group of compounds
17β-estradiol,
ethinylestradiol and
17β-estradiol valerate
and the progestogen is selected from the group of compounds
gestodene,
levonorgestrel,
desogestrel,
3-ketodesogestrel,
drospironenone,
cyproterone acetate,
norgestimate and
norethisterone and
the estrogen of the second hormone component is selected from the group of compounds
17β-estradiol,
ethinylestradiol and
17β-estradiol valerate.

3. Combination preparation according to Claim 2, **characterized in that** the estrogen of the first hormone component is contained in. each daily dosage unit in a dose of
1.0 to 6.0 mg.of 17β-estradiol,
0.015 to 0.025 mg of ethinylestradiol, and
1.0 to 4.0 mg of 17β-estradiol valerate
and the progestogen is contained in each daily dosage unit in a dose of
0.05 to 0.075 mg of gestodene,
0.05 to 0.125 mg of levonorgestrel,
0.06 to 0.15 mg of desogestrel,
0.06 to 0.15 mg of 3-ketodesogestrel,
1.0 to 3.0 mg of drospironenone,
1.0 to 2.0 mg of cyproterone acetate,
0.2 mg to 0.3 mg of norgestimate and
0.35 to 0.75 mg of norethisterone.

4. Combination preparation according to Claim 2 or 3, **characterized in that** the second hormone component contains, in each daily dosage unit, an amount of:
1.0 to 6.0 mg of 17β-estradiol,
0.002 to 0.04 mg of ethinylestradiol, and
1.0 to 4.0 mg of 17β-estradiol valerate.

5. Combination preparation according to Claim 4, **characterized in that** the second hormone component in each daily dosage unit contains ethinylestradiol in an amount of 0.01 to 0.025 mg.

6. Combination preparation according to one of the preceding claims, **characterized in that** the total number of hormone daily units is 28.

## Revendications

1. Préparation pharmaceutique combinée comprenant deux composants hormonaux confectionnés séparément dans l'espace dans une unité d'emballage, destinés à une administration orale séquentielle dans le temps, constitués à chaque fois d'un certain nombre d'unités de dosage journalières logées séparément dans l'espace et de manière à pouvoir être prélevées individuellement dans l'unité d'emballage, un premier des composants hormonaux contenant comme agent actif hormonal en combinaison une préparation d'estrogène et de gestagène en un dosage au moins suffisant pour arrêter l'ovulation, en une exécution soit en une, soit en plusieurs phases et le deuxième composant hormonal contenant comme agent actif hormonal seulement une préparation d'estrogène, le premier composant hormonal comprenant 24 unités journalières et le deuxième composant hormonal 4 à 10 unités journalières, les unités journalières du premier composant hormonal ne contenant pas la combinaison d'un estrogène biogène et d'un estrogène synthétique et le nombre total d'unités journalières hormonales étant égal au nombre total de jours du cycle souhaité, mais au minium de 28 jours.

2. Préparation combinée selon la revendication 1, **caractérisée en ce que** l'estrogène du premier composant hormonal est choisi parmi le groupe des composés
17β-estradiol
éthinylestradiol et
valérate de 17β-estradiol
et le gestagène parmi le groupe de composés
gestodène
levonorgestrel
desogestrel
3-cétodesogestrel
drospironenone
acétate de cyproterone
norgestimate et
norethisterone et
l'estrogène du deuxième composant hormonal est choisi parmi le groupe des composés
17β-estradiol
éthinylestradiol et
valérate de 17β-estradiol.

3. Préparation combinée selon la revendication 2, **caractérisée en ce que** l'estrogène du premier composant hormonal est contenu dans chaque unité de dosage journalière en une dose de
1,0 à 6,0 mg de 17β-estradiol
0,015 à 0,025 mg d'éthinylestradiol
1,0 à 4,0 mg de valérate de 17β-estradiol
et le gestagène dans chaque unité de dosage journalière est contenu en une dose de
0,05 à 0,075 mg de gestodène
0,05 à 0,125 mg de levonorgestrel
0,06 à 0,15 mg de desogestrel
0,06 à 0,15 mg de 3-cétodesogestrel
1,0 à 3,0 mg de drospironenone
1,0 à 2,0 mg d'acétate de cyproterone
0,2 à 0,3 mg de norgestimate
0,35 à 0,75 mg de norethisterone.

4. Préparation combinée selon les revendications 2 ou 3, **caractérisée en ce que** dans le deuxième composant hormonal, dans chaque unité de dosage journalière est contenue une quantité de
1,0 à 6,0 mg de 17β-estradiol
0,002 à 0,04 mg d'éthinylestradiol et
1,0 à 4,0 mg de valérate de 17β-estradiol.

5. Préparation combinée selon la revendication 4, **caractérisée en ce que** dans le deuxième composant hormonal, dans chaque unité de dosage journalière, l'éthinylestradiol est contenu en une quantité de 0,01 à 0,025 mg.

6. Préparation combinée selon l'une quelconque des revendications précédentes, **caractérisée en ce que** le nombre total d'unités journalières hormonales est de 28.
